# EUROPEAN PATENT APPLICATION

(11) **EP 4 831 163 A1**
(43) Date of publication of application: **28.10.2026**
(21) Application number: 24908080.5
(22) Date of filing: 18.12.2024
(51) Int. Cl.: C07D 265/30, C07B 55/00, A61K 31/5375, A61P 25/00, A61P 25/24

(54) **IMPROVED METHOD FOR PREPARATION OF (S)-BILOXAZINE OR SALT THEREOF**

(30) Priority: 18.12.2023 KR 20230184986
(71) Applicant: Astrogen, Inc., Daegu 41072 (KR)
(72) Inventor: HWANG, Su-Kyeong, Daegu 41072 (KR); RYU, Hyung Chul, Daegu 41072 (KR)
(74) Representative: Dehns
(86) International application number: PCT/KR2024/020636
(87) International publication number: WO 2025/135797

(57) **Abstract**

The present disclosure relates to an improved method for preparing optically pure (S)-viloxazine or a salt thereof. Attention-deficit/hyperactivity disorder (ADHD) can be prevented or treated by selectively administering (S)-viloxazine prepared according to the preparation method of the present disclosure. Accordingly, the dosage of the drug can be reduced by half or more, and side effects can be reduced.

## Description

### Technical Field

The present disclosure relates to an improved method for preparing optically pure (S)-viloxazine or a salt thereof.

### Background Art

Viloxazine hydrochloride is a racemic compound and a drug that has long been used as an antidepressant in the UK. It was approved by the FDA as a therapeutic agent for attention-deficit/hyperactivity disorder (ADHD) in children in 2021 and as a therapeutic agent for ADHD in adults in 2022, and is sold under the brand name Qelbree as a non-stimulant therapeutic agent for ADHD. This drug modulates the activity of serotonin as a 5HT2B receptor antagonist and a 5-HT2C receptor agonist, and selectively inhibits norepinephrine reuptake. In addition, viloxazine hydrochloride is evaluated to have a low risk of drug abuse, dependence, or withdrawal symptoms upon treatment discontinuation due to its low effect on dopamine.

Viloxazine hydrochloride is provided in the form of extended-release capsules (100 mg, 200 mg, 400 mg) that can be taken once a day by patients with ADHD, is metabolized through the CYP2D6 enzyme, and the majority of its metabolites are excreted through urine. In clinical trials, patients with ADHD who took viloxazine hydrochloride showed improved symptoms compared to patients who took a placebo, and it was confirmed to be effective in alleviating the main symptoms of ADHD, such as inattention, hyperactivity, and impulsivity. In addition, some of the patients administered viloxazine hydrochloride experienced an increase in heart rate and blood pressure. The most common side effects include drowsiness, reduced appetite, fatigue, nausea, and insomnia, and there may be a risk of worsening symptoms in patients with bipolar disorder. There may be an increased risk of suicidal ideation or suicide during initial treatment or dosage adjustment.

On the other hand, viloxazine hydrochloride is known as a drug with a large difference in efficacy between the (R)-form and the (S)-form. The efficacy of the (S)-form is about 5 times higher than that of the (R)-form, and it is known that the (R)-form has no effect on ADHD upon long-term administration. In addition, generally, if the (R)-form non-selectively binds to non-target receptors or follows other metabolic pathways in the body, side effects may increase.

On the other hand, generally, chiral compounds are obtained by synthesizing a racemic compound and then separating each isomer through a chiral resolution process. At this time, since the inactive form must be discarded or reprocessed and converted into the active form, there is a problem in that the preparation requires a significant amount of cost and time.

To solve this problem, the present inventors synthesized the pure (S)-viloxazine free base of Formula 1 through Reaction Scheme 1 or 2 using the compound of Formula 2 as a starting material, and synthesized a novel salt of (S)-viloxazine through Reaction Scheme 3.

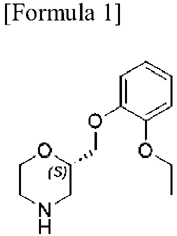

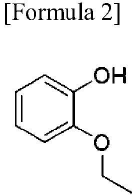

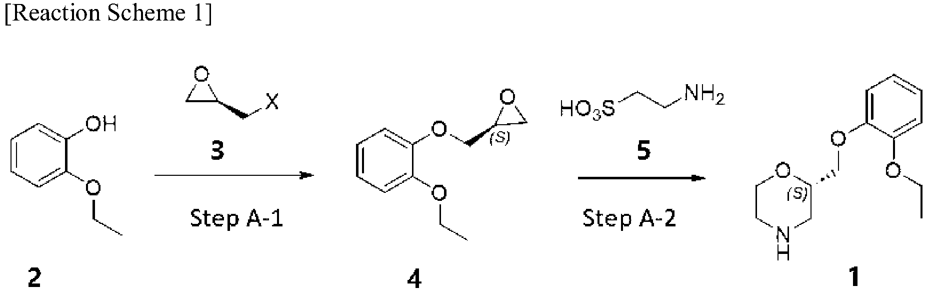

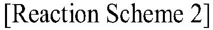

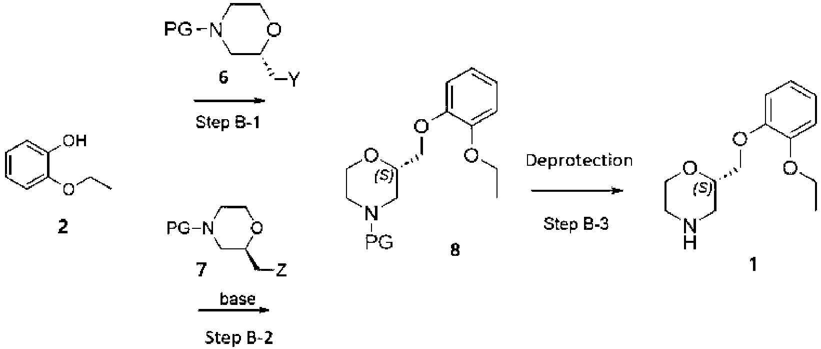

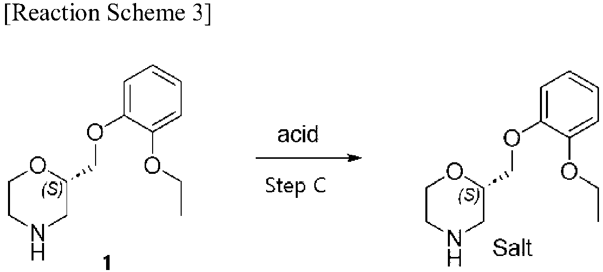

### Disclosure of Invention

### Technical Problem

According to the preparation method of the present disclosure, optically pure (S)-viloxazine or a salt thereof can be prepared by an improved method.

In addition, an object of the present disclosure is to use (S)-viloxazine or a novel salt thereof prepared according to the present disclosure for the prevention or treatment of attention-deficit/hyperactivity disorder (ADHD).

### Solution to Problem

[1] In one aspect, the present disclosure relates to a method for preparing a compound of Formula 1 or a salt thereof, the method comprising: reacting a compound of Formula 2 with a compound of Formula 3 in the presence of a base and a solvent (Step A-1); and reacting a compound of Formula 4 obtained from Step A-1 with a compound of Formula 5 in the presence of a base and a solvent (Step A-2), 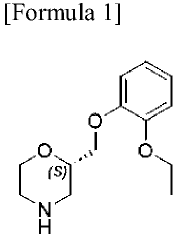 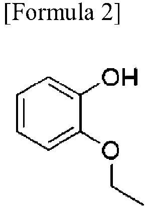 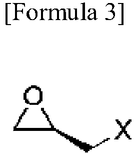 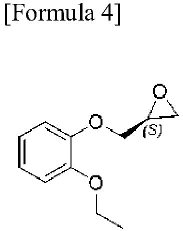 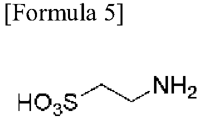 wherein X is Cl, Br, I, OMs (O-methane sulfonyl), or OTs (O-Tosyl) in Formula 3.
[2] In another aspect, the present disclosure relates to a method for preparing a compound of Formula 1 or a salt thereof, comprising: reacting a compound of Formula 2 with (i) a compound of Formula 6 in the presence of a solvent (step B-1) or (ii) a compound of Formula 7 in the presence of a base and a solvent (step B-2); and deprotecting a compound of Formula 8 obtained from step B-1 or step B-2 (step B-3), 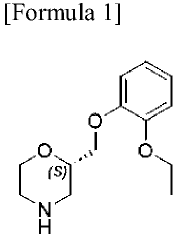 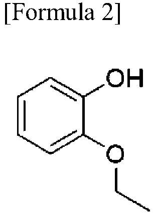 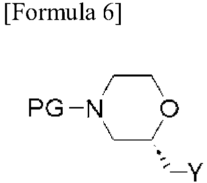 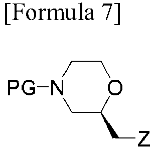 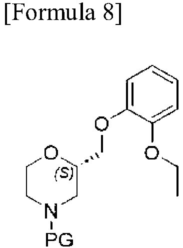
   wherein PG is a protecting group in Formulas 6 to 8,
   Y is OH in Formula 6, and
   Z is Cl, Br, I, OMs (O-methane sulfonyl), or OTs (O-Tosyl) in Formula 7.
[3] In [1] above, the compound of Formula 3 in step A-1 may be used in an amount of 0.90 to 2.0 equivalents with respect to the compound of Formula 2.
[4] In [1] above, the base in step A-1 may be at least one selected from the group consisting of sodium hydroxide, potassium hydroxide, calcium hydroxide, triethylamine, diethylamine, sodium carbonate, potassium carbonate, sodium bicarbonate, potassium bicarbonate, sodium t-butoxide, potassium t-butoxide, sodium hydride, sodium acetate, potassium acetate, and mixtures thereof.
[5] In [1] above, the base in step A-1 may be used in an amount of 0.9 to 2.0 equivalents with respect to the compound of Formula 2.
[6] In [1] above, the solvent in step A-1 may be at least one selected from the group consisting of ethyl acetate, dichloromethane, toluene, methyl-t-butyl ether, acetone, tetrahydrofuran, dimethylformamide, N-methylpyrrolidone, dimethylacetamide, acetonitrile, and diethyl ether.
[7] In [1], the solvent in step A-1 may be used in a weight amount of 6 to 20 times the weight of the compound of Formula 2.
[8] In [1], the base in step A-2 may be any one or more selected from the group consisting of sodium hydroxide, potassium hydroxide, calcium hydroxide, triethylamine, diethylamine, sodium carbonate, potassium carbonate, sodium bicarbonate, potassium bicarbonate, sodium t-butoxide, potassium t-butoxide, sodium hydride, sodium acetate, potassium acetate, and mixtures thereof.
[9] In [1], the solvent in step A-2 may be any one or more selected from the group consisting of purified water, methanol, ethanol, isopropanol, butanol, and hexanol.
[10] In [1], the solvent in step A-2 may be used in a weight amount of 6 to 20 times the weight of the compound of Formula 5.
[11] In [2], the protecting group in step B-1 and step B-2 may be tert-butoxycarbonyl (Boc), benzyl carbonyl (CBz), 9-fluorenylmethoxycarbonyl (Fmoc), or benzyl (Bn).
[12] In [2], the solvent in step B-1 may be at least one selected from the group consisting of tetrahydrofuran, benzene, dichloromethane, toluene, xylene, acetonitrile, and ethyl acetate.
[13] In [2], the base in step B-2 may be at least one selected from the group consisting of sodium hydroxide, potassium hydroxide, calcium hydroxide, triethylamine, diethylamine, sodium carbonate, potassium carbonate, sodium bicarbonate, potassium bicarbonate, sodium t-butoxide, potassium t-butoxide, sodium hydride, sodium acetate, potassium acetate, and a mixture thereof.
[14] In [2], the solvent in step B-2 may be at least one selected from the group consisting of ethyl acetate, dichloromethane, toluene, methyl-t-butyl ether, acetone, tetrahydrofuran, dimethylformamide, N-methylpyrrolidone, dimethylacetamide, acetonitrile, and diethyl ether.
[15] In [1] or [2], a step of reacting the prepared compound of Formula 1 with an acid in a solvent to form a salt of the compound of Formula 1 may be further included.
[16] In [15], the solvent may be at least one selected from the group consisting of ethyl acetate, dichloromethane, toluene, methyl-t-butyl ether, acetone, tetrahydrofuran, n-heptane, and diethyl ether.
[17] In [15], the acid may be at least one selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methanesulfonic acid, ethanesulfonic acid, benzenesulfonic acid, and toluenesulfonic acid.
[18] In [15] above, the acid may be hydrochloric acid or acetic acid.
[19] In [15] above, the salt of the compound of Formula 1 obtained may be any one selected from the group consisting of (S)-viloxazine hydrochloride, (S)-viloxazine hydrobromide, (S)-viloxazine sulfate, (S)-viloxazine phosphate, (S)-viloxazine nitrate, (S)-viloxazine acetate, (S)-viloxazine glycolate, (S)-viloxazine lactate, (S)-viloxazine pyruvate, (S)-viloxazine malonate, (S)-viloxazine succinate, (S)-viloxazine glutarate, (S)-viloxazine fumarate, (S)-viloxazine malate, (S)-viloxazine mandelate, (S)-viloxazine tartrate, (S)-viloxazine citrate, (S)-viloxazine ascorbate, (S)-viloxazine palmitate, (S)-viloxazine maleate, (S)-viloxazine benzoate, (S)-viloxazine hydroxybenzoate, (S)-viloxazine phenylacetate, (S)-viloxazine cinnamate, (S)-viloxazine salicylate, (S)-viloxazine methanesulfonate, (S)-viloxazine ethanesulfonate, (S)-viloxazine benzenesulfonate, and (S)-viloxazine toluenesulfonate.
[20] In [15], the salt of the compound of Formula 1 obtained may be (S)-viloxazine hydrochloride or (S)-viloxazine acetate.
[21] In another aspect, the present disclosure relates to a pharmaceutical composition for treating or preventing attention-deficit/hyperactivity disorder (ADHD), comprising the compound of Formula 1 or a salt thereof prepared according to [1], [2], or [15].
[22] In [21], the pharmaceutical composition may be administered by an oral, parenteral, or topical administration route.
[23] In another aspect, the present disclosure relates to a pharmaceutical composition for treating or preventing attention-deficit/hyperactivity disorder (ADHD), comprising (S)-viloxazine acetate.
[24] In another aspect, the present disclosure relates to a method for preventing or treating attention-deficit/hyperactivity disorder in a subject, comprising administering to the subject the compound of Formula 1 or a salt thereof prepared according to [1], [2], or [15].
[25] In another aspect, the present disclosure relates to the use of the compound of Formula 1 or a salt thereof prepared according to [1], [2] or [15] for the preparation of a medicament for preventing or treating attention-deficit/hyperactivity disorder.
[26] In another aspect, the present disclosure relates to the use of the compound of Formula 1 or a salt thereof prepared according to [1], [2] or [15] for preventing or treating attention-deficit/hyperactivity disorder.
[27] In another aspect, the present disclosure relates to a method for preventing or treating attention-deficit/hyperactivity disorder in a subject, the method comprising administering (S)-viloxazine acetate to the subject.
[28] In another aspect, the present disclosure relates to the use of (S)-viloxazine acetate for the preparation of a medicament for preventing or treating attention-deficit/hyperactivity disorder.
[29] In another aspect, the present disclosure relates to the use of (S)-viloxazine acetate for preventing or treating attention-deficit/hyperactivity disorder.

### Advantageous Effects of Invention

The preparation method of the present disclosure is economical, and attention-deficit/hyperactivity disorder (ADHD) can be prevented or treated by selectively administering (S)-viloxazine or a salt thereof prepared according to the preparation method of the present disclosure. Accordingly, the dosage of the drug can be reduced by more than half, and side effects can be reduced.

In addition, (S)-viloxazine or a novel salt thereof prepared according to the preparation method of the present disclosure has excellent norepinephrine reuptake inhibition efficacy and excellent thermal stability.

### Brief Description of Drawings

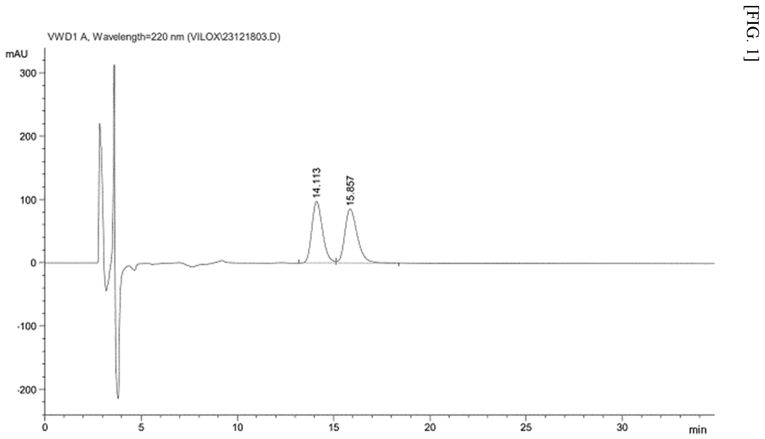

FIG. 1 shows an analysis of (R,S)-viloxazine hydrochloride of Comparative Example 1 by chiral HPLC.

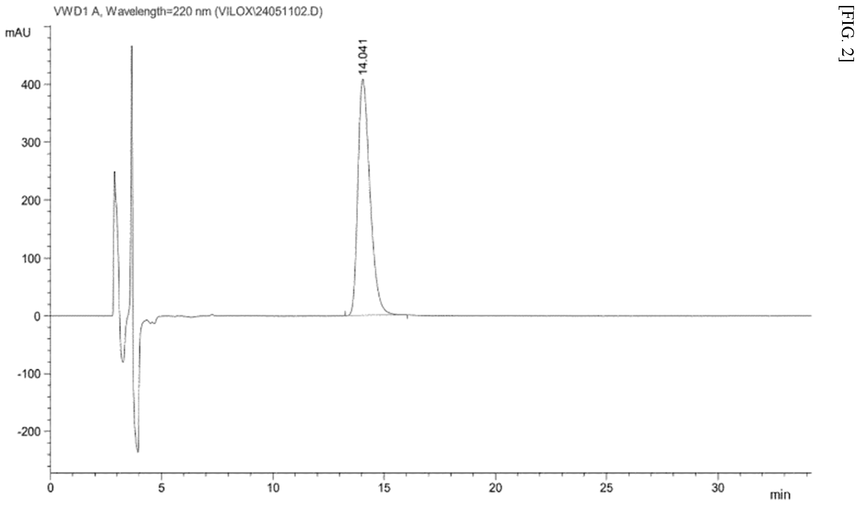

FIG. 2 shows an analysis of (S)-viloxazine hydrochloride of Example 6 by chiral HPLC.

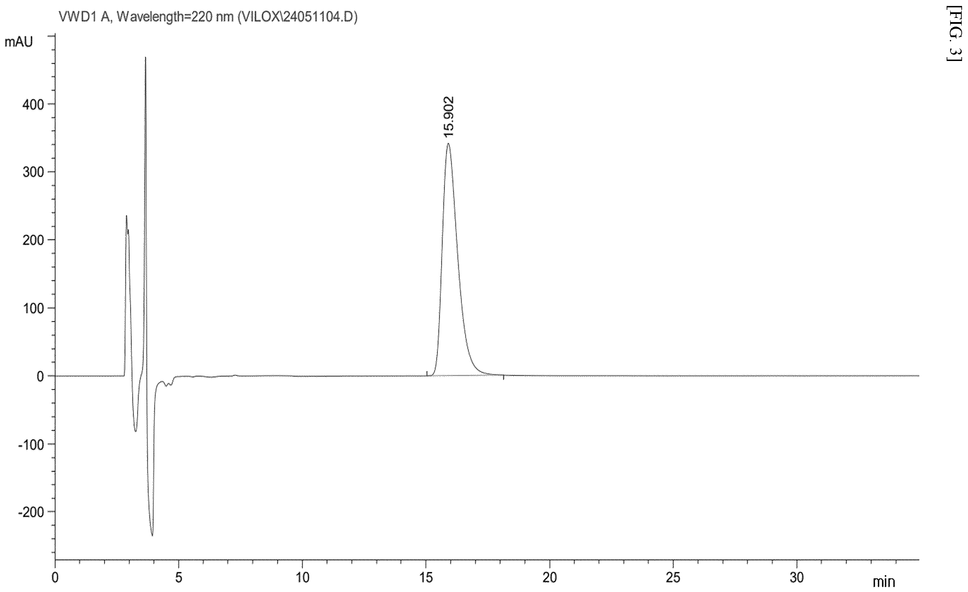

FIG. 3 shows an analysis of (R)-viloxazine hydrochloride of Comparative Example 2 by chiral HPLC.
FIG. 4 shows a measurement of the norepinephrine reuptake inhibition rate (IC₅₀) of viloxazine hydrochloride of Example 6 and Comparative Examples 1 and 2.
FIG. 5 shows a measurement of the norepinephrine reuptake inhibition rate (IC₅₀) of the novel salts of viloxazine of Examples 6 to 16.

### Best Mode for Carrying out the Invention

The terminology used in this specification is used to properly express preferred embodiments of the present disclosure, and the definitions of these terms should be understood based on the description throughout this specification. Throughout the specification, when a part "comprises" a certain component, this means that it may further include other components, rather than excluding other components, unless there is a specific description to the contrary.

All technical terms used in the present disclosure are used in the same meaning as generally understood by a person of average skill in the relevant field of the present disclosure, unless otherwise defined. In addition, although preferred methods or samples are described in this specification, those similar or equivalent thereto are also included in the scope of the present disclosure.

In one aspect, the present disclosure relates to a method for preparing a compound of Formula 1 or a salt thereof from a compound of Formula 2, wherein the compound of Formula 2 is 2-ethoxyphenol, and the compound of Formula 1 is (S)-viloxazine free base.

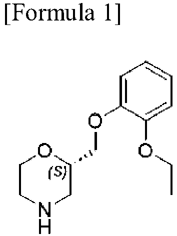

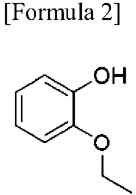

Specifically, the present disclosure relates to a method for preparing a compound of Formula 1 or a salt thereof, the method comprising:
reacting a compound of Formula 2 with a compound of Formula 3 in the presence of a base and a solvent (Step A-1); and
reacting a compound of Formula 4 obtained from Step A-1 with a compound of Formula 5 in the presence of a base and a solvent (Step A-2), 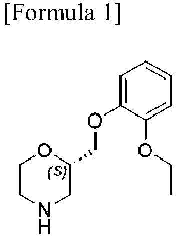 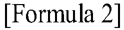 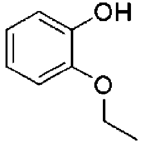 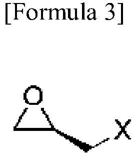 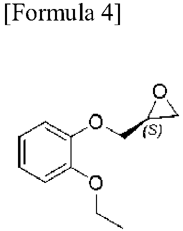 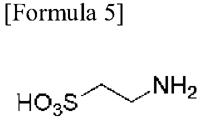
wherein X in Formula 3 is Cl, Br, I, OMs (O-methane sulfonyl), or OTs (O-Tosyl).

In the present disclosure, Step A-1 and Step A-2 may be according to the following Reaction Scheme 1.

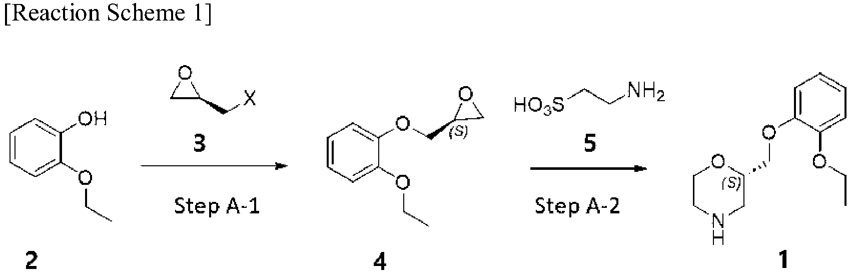

In the present disclosure, Step A-1 is a step of reacting the compound of Formula 2 with the compound of Formula 3 in the presence of a base and a solvent.

The compound of Formula 3 is an (R)-form oxirane derivative, and X in Formula 3 may be Cl, Br, I, OMs (O-methane sulfonyl), or OTs (O-Tosyl).

The compound of Formula 3 may be used in an amount of about 0.90 to 2.0 equivalents, about 0.95 to 1.5 equivalents, or about 0.95 to 1.3 equivalents, and preferably about 0.95 to 1.1 equivalents, with respect to the compound of Formula 2, but is not limited thereto.

In step A-1, the base may be one or more selected from the group consisting of sodium hydroxide, potassium hydroxide, calcium hydroxide, triethylamine, diethylamine, sodium carbonate, potassium carbonate, sodium bicarbonate, potassium bicarbonate, sodium t-butoxide, potassium t-butoxide, sodium hydride, sodium acetate, potassium acetate, and mixtures thereof. The base may be used in an amount of 0.90 to 2.0 equivalents, about 0.95 to 1.5 equivalents, about 0.95 to 1.4 equivalents, or about 0.95 to 1.3 equivalents, and preferably about 0.95 to 1.2 equivalents, with respect to the compound of Formula 2, but is not limited thereto.

In step A-1, the solvent may be one or more selected from the group consisting of ethyl acetate, dichloromethane, toluene, methyl-t-butyl ether, acetone, tetrahydrofuran, dimethylformamide, N-methylpyrrolidone, dimethylacetamide, acetonitrile, and diethyl ether, and may preferably be ethyl acetate, methyl-t-butyl ether, or dimethylformamide. The amount of the solvent used may be about 6 to 20 times, preferably about 5 to 15 times, and most preferably about 4 to 12 times the weight of the compound of Formula 2, but is not limited thereto.

The reaction time of step A-1 may be about 1 to 24 hours, preferably 3 to 12 hours, and most preferably 4 to 8 hours.

The reaction temperature of step A-1 is about 25 °C to 80 °C, preferably about 30 to 70 °C, and most preferably about 40 to 60 °C. At a temperature of about 80 °C or higher, side reactions may occur.

In the present disclosure, step A-2 is a step of reacting the compound of Formula 4 obtained from step A-1 with the compound of Formula 5 in the presence of a base and a solvent. The compound of Formula 5 is ethanolamine-O-sulphate, and as a result of the reaction in step A-2, (S)-viloxazine free base is synthesized.

The base in step A-2 may be one or more selected from the group consisting of sodium hydroxide, potassium hydroxide, calcium hydroxide, triethylamine, diethylamine, sodium carbonate, potassium carbonate, sodium bicarbonate, potassium bicarbonate, sodium t-butoxide, potassium t-butoxide, sodium hydride, sodium acetate, potassium acetate, and mixtures thereof, and it may be used by being appropriately diluted in purified water.

The solvent of step A-2 may be one or more selected from the group consisting of purified water, methanol, ethanol, isopropanol, butanol, and hexanol, is preferably methanol, ethanol, or isopropanol, and is most preferably methanol. The amount of the solvent used is 6 to 20 times, preferably 5 to 15 times, and most preferably 4 to 12 times the weight of the compound of Formula 5, but is not limited thereto. In addition, when using the organic solvent, 2 to 15% of water based on the total amount of the solvent used may be mixed for smooth stirring.

The reaction time of step A-2 may be 1 to 12 hours, preferably 2 to 10 hours, and most preferably 3 to 6 hours.

The reaction temperature of step A-2 is from 40°C to a reflux temperature, and if the temperature is too low, the yield of the reaction decreases.

In another aspect, the present disclosure relates to a method for preparing a compound of Formula 1 or a salt thereof, the method comprising: (i) reacting a compound of Formula 2 with a compound of Formula 6 in the presence of a solvent (step B-1) or (ii) reacting a compound of Formula 2 with a compound of Formula 7 in the presence of a base and a solvent (step B-2); and deprotecting a compound of Formula 8 obtained from step B-1 or step B-2 (step B-3), 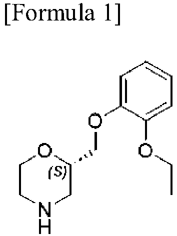 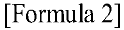 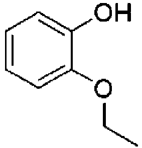 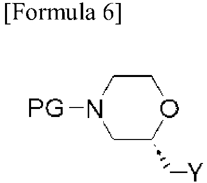 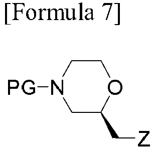 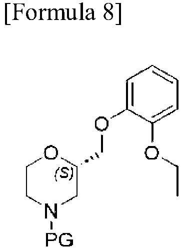
wherein PG is a protecting group in Formulas 6 to 8,
Y is OH in Formula 6, and
Z is Cl, Br, I, OMs (O-methane sulfonyl), or OTs (O-Tosyl) in Formula 7.

In the present disclosure, the steps B-1, B-2, and B-3 may be Reaction Scheme 2 below.

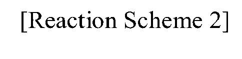

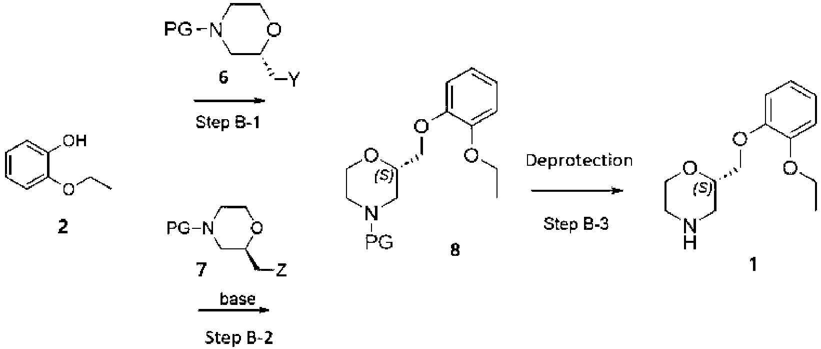

In the present disclosure, the step B-1 is a step of reacting the compound of Formula 2 with the compound of Formula 6 in the presence of a solvent.

PG in Formulas 6 to 8 is a protecting group, and may be, for example, tert-butoxycarbonyl (Boc), benzyl carbonyl (CBz), 9-fluorenylmethoxycarbonyl (Fmoc), or benzyl (Bn). On the other hand, Y is OH in Formula 6.

In the step B-1, the solvent may be one or more selected from the group consisting of tetrahydrofuran, benzene, dichloromethane, toluene, xylene, acetonitrile, and ethyl acetate.

In addition, in the step B-1, an alcohol (e.g., 2-ethoxyphenol) and triphenylphosphine or tributylphosphine, or the like may be used, and they combine to form a phosphonium intermediate. The amount used is 0.8 to 2.0 equivalents with respect to Formula 2, preferably 1.0 to 1.8 equivalents, and most preferably 1.2 to 1.5 equivalents.

In addition, in step B-1, DEAD (diethyl azodicarboxylate), DIAD (diisopropyl azodicarboxylate), DBAD (dibenzyl azodicarboxylate), and the like may be used as an oxidizing agent for removing the hydrogen of an alcohol. The amount used is 0.8 to 2.0 equivalents with respect to the compound of Formula 2, preferably 1.0 to 1.8 equivalents, and most preferably 1.2 to 1.5 equivalents.

In the present disclosure, step B-2 is a step of reacting the compound of Formula 2 with the compound of Formula 7 in the presence of a base and a solvent. Z in Formula 7 may be Cl, Br, I, OMs (O-methane sulfonyl), or OTs (O-Tosyl).

The base in step B-2 may be at least one selected from the group consisting of sodium hydroxide, potassium hydroxide, calcium hydroxide, triethylamine, diethylamine, sodium carbonate, potassium carbonate, sodium bicarbonate, potassium bicarbonate, sodium t-butoxide, potassium t-butoxide, sodium hydride, sodium acetate, potassium acetate, and mixtures thereof, and may be used after being appropriately diluted in purified water.

In step B-2, the solvent may be one or more selected from the group consisting of ethyl acetate, dichloromethane, toluene, methyl-t-butyl ether, acetone, tetrahydrofuran, dimethylformamide, N-methylpyrrolidone, dimethylacetamide, acetonitrile, and diethyl ether, and may preferably be ethyl acetate, methyl-t-butyl ether, acetonitrile, or dimethylformamide. The amount of the solvent used is about 6 to 20 times, preferably about 5 to 15 times, and most preferably about 4 to 12 times the weight of the compound of Formula 2, but is not limited thereto.

The reaction time of step B-1 and step B-2 may be 1 to 12 hours, preferably 2 to 10 hours, and most preferably 3 to 6 hours.

In the present disclosure, step B-3 is a step of deprotecting the compound of Formula 8 obtained from step B-1 and step B-2. As a result of the reaction in step B-3, (S)-viloxazine free base is synthesized.

The deprotection in step B-3 may be appropriately performed in consideration of the reaction conditions for removing the protecting group and the effect on functional groups other than the protecting group to be removed. For example, the tert-butoxycarbonyl (Boc) protecting group can be removed using trifluoroacetic acid (TFA), a strong acid (e.g., HCl), or a reducing agent such as lithium aluminum hydride (LAH). The benzyl carbonyl (CBz) protecting group can be removed by hydrogenolysis using a catalytic amount of palladium (Pd/C). The 9-fluorenylmethoxycarbonyl (Fmoc) protecting group can be removed using a solution of piperidine mixed with DMF. benzyl (Bn) can be removed by hydrochloric acid or hydrogenolysis.

The present disclosure may further include a step (step C) of forming a salt of the compound of Formula 1 by reacting the compound of Formula 1 prepared according to the present disclosure with an acid in a solvent.

The step C may be according to the following Reaction Scheme 3.

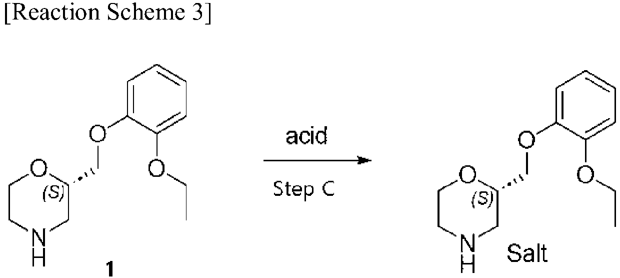

In step C, the solvent may be one or more selected from the group consisting of ethyl acetate, dichloromethane, toluene, methyl-t-butyl ether, acetone, tetrahydrofuran, n-heptane, and diethyl ether, and may preferably be ethyl acetate or methyl-t-butyl ether. The amount of the solvent used may be 6 to 20 times, preferably 5 to 15 times, and most preferably 4 to 12 times the weight of the compound of Formula 1, but is not limited thereto.

In step C, the acid may be a pharmaceutically usable inorganic acid or organic acid. For example, it may be at least one selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methanesulfonic acid, ethanesulfonic acid, benzenesulfonic acid, and toluenesulfonic acid.

In an embodiment, the acid in step C may be hydrochloric acid or acetic acid.

The salt of the compound of Formula 1 prepared according to step C is a novel salt of (S)-viloxazine.

In the present disclosure, the salt of the compound of Formula 1 to be obtained may be, for example, any one selected from the group consisting of (S)-viloxazine hydrochloride, (S)-viloxazine hydrobromide, (S)-viloxazine sulfate, (S)-viloxazine phosphate, (S)-viloxazine nitrate, (S)-viloxazine acetate, (S)-viloxazine glycolate, (S)-viloxazine lactate, (S)-viloxazine pyruvate, (S)-viloxazine malonate, (S)-viloxazine succinate, (S)-viloxazine glutarate, (S)-viloxazine fumarate, (S)-viloxazine malate, (S)-viloxazine mandelate, (S)-viloxazine tartrate, (S)-viloxazine citrate, (S)-viloxazine ascorbate, (S)-viloxazine palmitate, (S)-viloxazine maleate, (S)-viloxazine benzoate, (S)-viloxazine hydroxybenzoate, (S)-viloxazine phenylacetate, (S)-viloxazine cinnamate, (S)-viloxazine salicylate, (S)-viloxazine methanesulfonate, (S)-viloxazine ethanesulfonate, (S)-viloxazine benzenesulfonate, and (S)-viloxazine toluenesulfonate.

In an embodiment, the salt of the compound of Formula 1 obtained may be (S)-viloxazine hydrochloride or (S)-viloxazine acetate.

The reaction time of step C may be 1 to 12 hours, preferably 2 to 10 hours, and most preferably 3 to 6 hours.

The reaction temperature of step C is about -10°C to 60°C, and if the temperature is too high, racemization occurs and the yield of the reaction is lowered.

In another aspect, the present disclosure relates to a pharmaceutical composition for treating or preventing attention-deficit/hyperactivity disorder (ADHD), comprising the compound of Formula 1 or a salt thereof prepared according to the preparation method.

The pharmaceutical composition comprising the compound of Formula 1 or a salt thereof has an excellent effect of selectively inhibiting norepinephrine reuptake.

In the present disclosure, "treatment" refers to any act in which the symptoms of a disease are improved or beneficially changed by the administration of a composition according to the present disclosure, "prevention" refers to any act of inhibiting or delaying a disease by the administration of the composition according to the present disclosure, and "improvement" means any act of ameliorating a bad condition of a disease by administering or ingesting the composition of the present disclosure to a subject.

A subject disease for the "treatment", "prevention", or "improvement" of the present disclosure may be attention-deficit/hyperactivity disorder (ADHD).

For the treatment of the above-described disease or condition, the pharmaceutical composition described in this specification may be administered as follows.

### Oral administration

The compound of the present disclosure may be administered orally. The oral cavity is a concept that includes swallowing. By oral administration, the compound of the present disclosure may enter the gastrointestinal tract or, for example, be directly absorbed from the mouth into the bloodstream, such as by buccal or sublingual administration.

A suitable composition for oral administration may be in a solid, liquid, gel, or powder form, and may have a formulation such as a tablet, lozenge, capsule, granules, or powders.

A composition for oral administration may optionally be enteric coated, and through the enteric coating, may exhibit delayed or sustained release. That is, a composition for oral administration according to the present disclosure may be a formulation having an immediate or modified release pattern.

Liquid formulations may include solutions, syrups, and suspensions, and such liquid compositions may be contained in soft or hard capsules. These formulations may include a pharmaceutically acceptable carrier, for example, water, ethanol, polyethylene glycol, cellulose, or oil. The formulation may also include one or more emulsifying agents and/or suspending agents.

In a tablet formulation, the amount of the drug, which is the active ingredient, may be present in an amount of about 0.05 wt% to about 95 wt% based on the total weight of the tablet, and more generally about 2 wt% to about 50 wt% of the formulation. In addition, the tablet may contain a disintegrant in an amount of about 0.5 wt% to about 35 wt%, and more generally about 2 wt% to about 25 wt% of the formulation. Examples of the disintegrant may include, but are not limited to, lactose, starch, sodium starch glycolate, crospovidone, croscarmellose sodium, maltodextrin, or a mixture thereof.

Suitable lubricants included for preparing a tablet may be present in an amount of about 0.1 wt% to about 5 wt%, and talc, silicon dioxide, stearic acid, calcium, zinc or magnesium stearate, sodium stearyl fumarate, or the like may be used as the lubricant, but are not limited thereto.

As a binder for preparing a tablet, gelatin, polyethylene glycol, sugar, gum, starch, polyvinylpyrrolidone, hydroxypropyl cellulose, hydroxypropyl methylcellulose, or the like may be used, and as a suitable diluent for preparing a tablet, mannitol, xylitol, lactose, dextrose, sucrose, sorbitol, starch, microcrystalline cellulose, or the like may be used, but are not limited thereto.

A solubilizer that may be optionally included in the tablet may be used in an amount of about 0.1 wt% to about 3 wt% based on the total weight of the tablet, and for example, polysorbate, sodium lauryl sulfate, sodium dodecyl sulfate, propylene carbonate, diethylene glycol monoethyl ether, dimethyl isosorbide, polyoxyethylene glycolized natural or hydrogenated castor oil, HCOR^{™} (Nikkol), oleyl ester, Gelucire^{™}, caprylic/capric mono/diglycerides, sorbitan fatty acid esters, Solutol HS^{™}, or the like may be used in the pharmaceutical composition according to the present disclosure, but is not limited thereto.

### Parenteral Administration

The compound of the present disclosure may be directly administered into the bloodstream, muscle, or internal organs. Suitable methods for parenteral administration include intravenous, intra-muscular, subcutaneous intraarterial, intraperitoneal, intrathecal, and intracranial injections. Suitable devices for parenteral administration include an injector including a syringe with a needle and a needle-free syringe, and an infusion method.

A composition for parenteral administration may be a formulation having an immediate or modified release pattern, and the modified release pattern may be a delayed or sustained release pattern.

The majority of parenteral formulations are liquid compositions, and these liquid compositions are aqueous solutions comprising an active ingredient according to the present disclosure, a salt, a buffer, an isotonic agent, and the like.

Parenteral formulations may also be prepared in a dried form (e.g., lyophilized) or as a sterile non-aqueous solution. These formulations may be used with a suitable vehicle such as sterile water. Solubility-enhancing agents may also be used in the preparation of a parenteral solution.

### Topical Administration

The compound of the present disclosure may be administered topically to the skin or transdermally. A formulation for the topical administration may include a lotion, a solution, a cream, a gel, a hydrogel, an ointment, a foam, an implant, a patch, and the like. A pharmaceutically acceptable carrier for the topical administration formulation may include water, alcohol, mineral oil, glycerin, polyethylene glycol, and the like. The topical administration may also be performed by, but is not limited to, electroporation, iontophoresis, phonophoresis, and the like.

The composition for topical administration may be a formulation having an immediate or modified release pattern, and the modified release pattern may be a delayed or sustained release pattern.

The pharmaceutical composition of the present disclosure may be administered in a therapeutically effective amount of the active ingredient. The therapeutically effective amount refers to a drug dosage that exhibits an effective preventive or therapeutic effect for attention-deficit/hyperactivity disorder (ADHD). A suitable total daily usage amount may be determined by a physician within the scope of sound medical judgment. A specific therapeutically effective amount for a particular patient may be applied differently depending on various factors and similar factors well known in the medical field, including the type and degree of the response to be achieved, the specific composition including the type and amount of the drug administered in combination and whether other agents are used, the age, weight, general health condition, sex, and diet of the patient, the time of administration, the route of administration, and the duration of treatment.

In another aspect, the present disclosure relates to a pharmaceutical composition for treating or preventing attention-deficit/hyperactivity disorder (ADHD), comprising (S)-viloxazine acetate.

In another aspect, the present disclosure relates to a method for preventing or treating attention-deficit/hyperactivity disorder (ADHD) of a subject, comprising administering to the subject the compound of Formula 1 or a salt thereof prepared according to the preparation method.

In the present disclosure, the term "subject" is used interchangeably with "individual," and the subject may be a mammal, for example, a human, rat, cow, horse, pig, dog, sheep, goat, or cat.

Overlapping descriptions with the aforementioned pharmaceutical composition of the present disclosure are omitted.

In another aspect, the present disclosure relates to a method for preventing or treating attention-deficit/hyperactivity disorder of a subject, comprising administering (S)-viloxazine acetate to a subject.

In another aspect, the present disclosure relates to the use of the compound of Formula 1 or a salt thereof prepared according to the preparation method, for preparing a medicament for preventing or treating attention-deficit/hyperactivity disorder (ADHD) in a subject.

Overlapping descriptions with the above-described pharmaceutical composition of the present disclosure are omitted.

In another aspect, the present disclosure relates to the use of (S)-viloxazine acetate for preparing a medicament for preventing or treating attention-deficit/hyperactivity disorder.

In another aspect, the present disclosure relates to the use of the compound of Formula 1 or a salt thereof prepared according to the preparation method, for preventing or treating attention-deficit/hyperactivity disorder (ADHD) in a subject.

Overlapping descriptions with the above-described pharmaceutical composition of the present disclosure are omitted.

In another aspect, the present disclosure relates to the use of (S)-viloxazine acetate for preventing or treating attention-deficit/hyperactivity disorder.

The matters mentioned in all compositions, treatment methods, and uses of the present disclosure are applied in the same manner as long as they do not contradict each other.

Hereinafter, the present disclosure will be described in detail with reference to embodiments, etc., to facilitate understanding of the present disclosure. However, the embodiments according to the present disclosure may be modified into various other forms, and the scope of the present disclosure should not be construed as being limited to the embodiments described below. The embodiments of the present disclosure are provided to more completely describe the present disclosure to a person having average knowledge in the field to which the present disclosure pertains.

### Mode for the Invention

### Examples

### Example 1. Synthesis of (S)-2-((2-ethoxyphenoxy)methyl)oxirane (Step A-1)

2-ethoxyphenol (13.8 g, purchased from Aldrich) was dissolved in dimethylformamide (120 ml, purchased from Daejung Chemicals & Metals), and (R)-2-(chloromethyl)oxirane (9.25 g, purchased from Aldrich) and triethylamine (10.1 g) were slowly added, and the mixture was stirred at 30°C for 6 hours. After reaction completion, the mixture was purified by silica gel chromatography using a mixed solvent of ethyl acetate:n-hex (4:10) as a developing solvent, and pure (S)-2-((2-ethoxyphenoxy)methyl)oxirane was obtained as a colorless liquid (15.53 g, yield 80%).

1H NMR δ (DMSO-d₆) 6.94 (m, 4H), 4.07 (m, 3H), 3.03 (m, 1H), 2.45 (m, 2H), 1.32 (t, 3H).

### Example 2. Synthesis of (S)-2-((2-ethoxyphenoxy)methyl)oxirane (Step A-1)

2-ethoxyphenol (13.8g, purchased from Aldrich) was dissolved in tetrahydrofuran (120ml, purchased from Daejung Chemicals & Metals), and (R)-2-(chloromethyl)oxirane (9.25g, purchased from Aldrich) and triethylamine (10.1g) were slowly added, and the mixture was stirred at 50°C for 6 hours. After reaction completion, purification by silica gel chromatography was performed using a mixed solvent of ethyl acetate:n-hex (4:10) as a developing solvent, and pure (S)-2-((2-ethoxyphenoxy)methyl)oxirane was obtained as a colorless liquid (12.23g, 64% yield).

1H NMR δ (DMSO-d₆) 6.94 (m, 4H), 4.07 (m, 3H), 3.03 (m, 1H), 2.45 (m, 2H), 1.32 (t, 3H).

### Example 3. Synthesis of (S)-viloxazine free base (Step A-2)

Ethanolamine-O-sulfate (17.5 g, purchased from EOS) was added to a mixture of (S)-2-((2-ethoxyphenoxy)methyl)oxirane of Example 2 (4 g), a 16 M NaOH solution (9.5 g), and methanol (6 ml), and the mixture was stirred at 40°C for 2 hours. To the mixture, solid NaOH (7.5 g) and toluene (26 ml) were added, and the reaction was allowed to proceed at 65°C for 7 hours. To the cooled mixture, 45 ml of water and 15 ml of toluene were added, the organic layer was separated, and the toluene was extracted with 2 N HCl, after which the acidic extract was alkalized to pH 11 using 4 M NaOH and extracted again with toluene. The toluene layer was washed with an aqueous solution of sodium chloride (NaCl), and after drying, was purified by silica gel chromatography using a mixed solvent of ethyl acetate:ethanol (4:0.1) as a developing solvent to obtain pure (S)-viloxazine (3.5 g, 19.8 mmol, 66% yield).

[α]D 20 -1.47 (c=1, MeOH), 1H NMR δ (DMSO-d₆) 6.94 (m, 4H), 3.97 (m, 7H), 3.13 (m, 4H), 1.32 (t, 3H).

### Example 4. Synthesis of (S)-viloxazine free base (Step A-2)

Ethanolamine-O-sulfate (17.5 g) was added to a mixture of (S)-2-((2-ethoxyphenoxy)methyl)oxirane of Example 2 (4 g, 29.9 mmol), an 8 M NaOH solution (19 g), and methanol (6 ml), and the mixture was stirred at 40°C for 2 hours. Solid NaOH (7.5 g) and toluene (26 ml) were added to the mixture, and the reaction was allowed to proceed at 65°C for 7 hours. To the cooled mixture, 45 ml of water and 15 ml of toluene were added, the organic layer was separated, and the toluene was extracted with 2 N HCl, and then the acidic extract was alkalized to pH 11 using 4 M NaOH and extracted again with toluene. The toluene layer was washed with an aqueous solution of sodium chloride (NaCl), and after drying, purified by silica gel chromatography using a mixed solvent of ethyl acetate:ethanol (4:0.1) as a developing solvent to obtain pure (S)-viloxazine free base (3.4 g, 60% yield).

[α]D 20 -1.47 (c=1, MeOH), 1H NMR δ (DMSO-d₆) 6.94 (m, 4H), 3.97 (m, 7H), 3.13 (m, 4H), 1.32 (t, 3H).

### Example 5-1. Synthesis of (S)-viloxazine free base (Steps B-1 and B-3)

10.0 g (46.03 mmol) of tert-butyl (S)-2-(hydroxymethyl)morpholine-4-carboxylate (purchased from Reliable Chem, China), 11.45 g (82.85 mmol) of 2-ethoxyphenol, 18.11 g (69.04 mmol) of triphenylphosphine (purchased from Daejung Chemicals & Metals), and 100 ml (10V) of ethyl acetate were placed in a reactor and cooled to 0 °C. Next, 12.10 g (59.83 mmol) of diisopropyl azodicarboxylate (purchased from Aldrich) was slowly added dropwise. The temperature was raised from 25 °C to 70 °C to allow the reaction to proceed. After reaction completion, 100 ml (10V) of water was added, the aqueous layer was removed, and then 20 g of MgSO₄ was added to the organic layer, and the mixture was filtered. To the filtered solution, HCl dissolved in isopropyl alcohol (IPA) was added, and the mixture was reacted for 24 hours at room temperature. After reaction completion, the mixture was neutralized with aqueous ammonia, the resulting solid was filtered, washed with isopropyl alcohol (IPA), and then concentrated under reduced pressure in a vacuum state, and purified by silica gel chromatography using a mixed solvent of ethyl acetate:ethanol (4:0.1) as a developing solvent to obtain pure (S)-viloxazine free base (7.64 g, 70% yield).

[α]D 20 -1.47 (c=1, MeOH), 1H NMR δ (DMSO-d₆) 6.94 (m, 4H), 3.97 (m, 7H), 3.13 (m, 4H), 1.32 (t, 3H).

### Example 5-2. Synthesis of (S)-viloxazine free base (Step B-2 and Step B-3)

28.0 g (100 mmol) of tert-butyl (R)-2-(bromomethyl)morpholine-4-carboxylate (purchased from Reliable Chem, China), 13.81 g (100 mmol) of 2-ethoxyphenol, 13.12 g (100 mmol) of potassium carbonate (purchased from Daejung Chemicals & Metals), and 280 ml (10V) of acetonitrile were placed in a reactor, heated to 60 °C, and stirred for 6 hours. After reaction completion, the mixture was concentrated under reduced pressure, 280 ml (10V) of water and 280 ml (10V) of ethyl acetate were added, the aqueous layer was removed, and then 30 g of MgSO₄ was added to the organic layer and filtered. To the filtered solution, HCl dissolved in isopropyl alcohol (IPA) was added, and the mixture was reacted at room temperature for 24 hours. After reaction completion, the mixture was neutralized with aqueous ammonia, and the resulting solid was filtered, washed with isopropyl alcohol (IPA), concentrated under reduced pressure in a vacuum state, and then purified by silica gel chromatography using a mixed solvent of ethyl acetate:ethanol (4:0.1) as a developing solvent to obtain pure (S)-viloxazine free base (18.6 g, yield 80%).

[α]D 20 -1.47 (c=1, MeOH), 1H NMR δ (DMSO-d₆) 6.94 (m, 4H), 3.97 (m, 7H), 3.13 (m, 4H), 1.32 (t, 3H).

### Example 6. Synthesis of (S)-viloxazine hydrochloride (Step C)

5 g of (S)-viloxazine free base obtained in Example 5-2 was added to 50 ml of ethyl acetate, and then 7 g (1 equivalent) of 3M HCl in IPA was slowly added dropwise. After stirring at room temperature for 1 to 2 hours, the mixture was filtered under reduced pressure and washed with 10 ml of ethyl acetate. (S)-viloxazine hydrochloride was obtained as a white solid by vacuum drying at 50°C (yield 83%).

Hereinafter, viloxazine salts were obtained in the same method by changing the type of acid (Examples 7 to 16 of Table 1).

Comparative Example 1. Synthesis of (R,S)-viloxazine hydrochloride (R,S)-viloxazine hydrochloride was synthesized in the same method as in Example 1, except that (R,S)-2-(chloromethyl)oxirane was used instead of (R)-2-(chloromethyl)oxirane.

### Comparative Example 2. Synthesis of (R)-viloxazine hydrochloride

(R)-viloxazine hydrochloride was synthesized in the same method as in Example 1, except that (S)-2-(chloromethyl)oxirane was used instead of (R)-2-(chloromethyl)oxirane.

The synthesized compounds are shown in Table 1.

**[Table 1]**

| Sample | Acid | Appearanc e | Yield | [α]D 20(c=1, MeOH) / ¹H NMR DMSO-d₆ | IC₅₀ (µM) |
|---|---|---|---|---|---|
| Comparative Example 1 | HCl | White solid | 51% | 0.32 / 9.69 (br s, 2H), 6.94 (m, 4H), 3.97 (m, 7H), 3.13 (m, 4H), 1.32 (t, 3H) | 62.9 |
| Comparative Example 2 | HCl | White solid | 43% | 4.92 / 9.70 (br s, 2H), 6.95 (m, 4H), 3.96 (m, 7H), 3.13 (m, 4H), 1.32 (t, 3H). | 108 |
| Example 6 | HCl | White solid | 83% | -4.51 / 9.69 (br s, 2H), 6.94 (m, 4H), 3.98 (m, 7H), 3.13 (m, 4H), 1.32 (t, 3H). | 22.5 |
| Example 7 | H₂SO₄ | White solid | 57% | -1.75 / 7.17 (m, 4H), 4.38 (m, 9H), 3.22 (m, 4H), 1.32 (t, 3H). | 29.3 |
| Example 8 | HBr | Light beige solid | 60% | -4.1 / 8.99 (br s, 2H), 6.93 (m, 4H), 4.06 (m, 7H), 3.11 (m, 4H), 1.32 (t, 3H). | 38.6 |
| Example 9 | HNO₃ | White solid | 45% | -3.56 / 8.88 (br s, 2H), 6.95 (m, 4H), 4.04 (m, 7H), 3.11 (m, 4H), 1.32 (t, 3H). | 42 |
| Example 10 | Acetic acid | White solid | 70% | - 3.47 / 6.90 (m, 4H), 5.22 (s, 2H), 3.91 (m, 7H), 2.66 (m, 4H), 1.89(s, 3H), 1.31 (t, 3H). | 24.3 |
| Example 11 | Trifluoro acetic acid | Light beige solid | 56% | -0.03 / 9.11 (br s, 2H), 6.94 (m, 4H), 4.04 (m, 7H), 3.02 (m, 4H), 1.32 (t, 3H). | 33.6 |
| Example 12 | Oxalic acid | White solid | 58% | -0.01 / 6.91 (m, 4H), 5.54 (s, 4H), 3.94 (m, 7H), 2.89 (m, 4H), 1.31 (t, 3H). | 34.3 |
| Example 13 | Fumaric acid | White solid | 66% | -1.62 / 6.92 (m, 4H), 6.49 (s, 1H), 5.23 (br s, 3H), 3.92 (m, 7H), 2.90 (m, 4H), 1.32 (t, 3H). | 36.1 |
| Example 14 | Maleic acid | Light beige solid | 55% | -3.60 / 6.95 (m, 4H), 6.02 (s, 1H), 3.98 (m, 7H), 2.90 (m, 4H), 1.32 (t, 3H). | 28.8 |
| Example 15 | Citric acid | White solid | 65% | -2.40 / 7.02 (m, 4H), 4.63 (s, 6H), 4.05 (m, 7H), 3.01 (m, 4H), 2.60(s, 4H), 1.41 (t, 3H). | 40.1 |
| Example 16 | Methane sulfonic acid | Light beige solid | 58% | -3.12 / 6.90 (m, 4H), 5.22 (s, 2H), 3.91 (m, 7H), 3.49(s, 3H), 2.66 (m, 4H), 1.31 (t, 3H). | 40.2 |

### Experimental Example 1. High-performance liquid chromatography (HPLC) analysis

HPLC analysis of the viloxazine hydrochloride of Example 6 and Comparative Examples 1 and 2 was performed under the following conditions. As a result, it was confirmed that the (S)-viloxazine hydrochloride of Example 6 was obtained with high purity.
1) Preparation of test solution
   50 mg of a sample was precisely weighed, placed in a 50 mL volumetric flask, ethanol was added to the mark, and dissolved by ultrasonication for 10 minutes to be used as a test solution.
2) HPLC conditions
   - Column: CHIRALPAK OD-H column (4.6 mm x 250 mm, 5 µm)
   - Mobile phase: A mixture of 2-propanol, heptane, diethylamine, and trifluoroacetic acid (150:850:1.5:2.0, v/v)
   - Flow rate: 1.0 mL/min
   - Detector: Ultraviolet absorbance spectrophotometer (230 nm)
   - Column temperature: 30 °C
   - Injection volume: 10 µL
   - Diluent: Ethanol
   - Analysis time: 35 minutes
3) Calculation $Amount of isomer \left(%\right) - \frac{{A}_{S}}{{A}_{R}} \times 100$

### Experimental Example 2: Measurement of norepinephrine reuptake inhibition rate (IC₅₀)

The norepinephrine reuptake ability in a cell line expressing the norepinephrine receptor was confirmed using a kit (Neurotransmitter Transporter Uptake Assay Kit, Molecular Devices) composed of a fluorescent material that mimics amine neurotransmitters and a masking dye.

First, HEK-293T cells stably expressing the norepinephrine receptor were seeded in a 96-well Black/Clear Bottom Plate to contain 6 x 10⁴ cells in 100 µl of cell culture medium per well. After incubating for 20 hours in a 37 °C, 5% CO₂ incubator to allow the cells to properly attach and stabilize on the plate, the cell culture medium was removed, and 100 µl of an HBSS+0.1% BSA with 20 mM HEPES aqueous solution in which a drug of a target concentration was dissolved was dispensed into each well. For the control group, 100 µl of a drug-free buffer solution (HBSS+0.1% BSA with 20 mM HEPES aqueous solution) was dispensed. The plate was incubated for 30 minutes in a 37 °C, 5% CO₂ incubator to allow the drug to sufficiently react with the receptor.

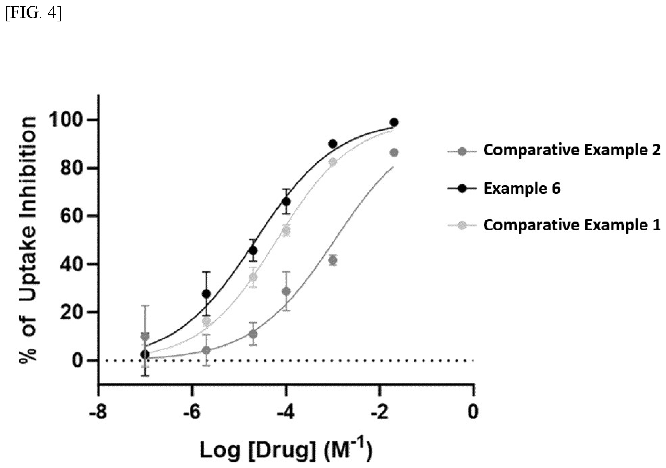

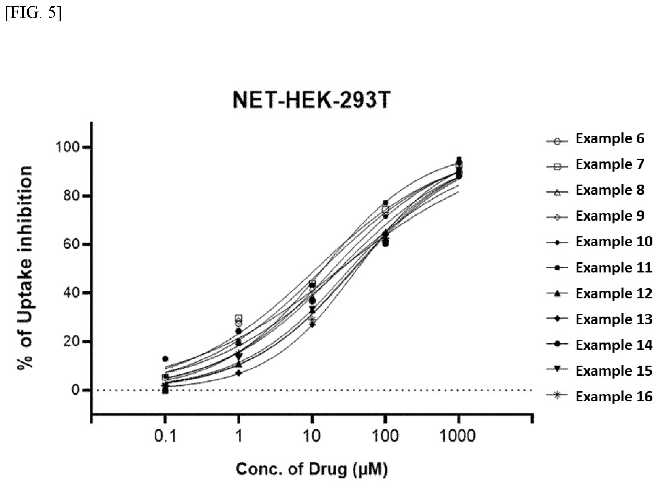

Thereafter, fluorescence was measured for a total of 30 minutes in Kinetic reading mode at Ex/Em=440 nm/520 nm using a fluorescence plate reader (SpectraMax^{®}). The measured fluorescence intensity value was converted into a value of "reuptake inhibition rate (%)" using the following formula, and then a dose-response curve was plotted through 4-parameter (Log) logistic regression, and the IC₅₀ value for each drug was calculated and compared (FIGS. 4 and 5). $\left[\frac{\left(Fluorescence intensity in the control group\right) - \left(Fluorescence intensity at each concentration\right)}{Fluorescence intensity in yhe control group}\times 100%\right]$

It was confirmed that the novel salts of (S)-viloxazine of Examples 6 to 16 exhibited superior inhibitory efficacy compared to the (R,S)-viloxazine hydrochloride of Comparative Example 1 or the (R)-viloxazine hydrochloride of Comparative Example 2.

### Experimental Example 3: Thermal stability test

8 mg each of the viloxazine hydrochloride of Comparative Examples 1 and 2, and the novel salts of (S)-viloxazine of Examples 6 to 16 were placed into opaque glass vials and stored at 40 ± 2 °C and 75 ± 5% RH. After 8 weeks, each sample was taken out, its content was analyzed by high-performance liquid chromatography (HPLC), and the presence of discoloration was visually confirmed (Table 2).

**[Table 2]**

| Sample | Acid used | Content after 0→8 weeks (%) | Discoloration |
|---|---|---|---|
| Comparative Example 1 | HCl | 99.5 → 98.7 | Discolored to an off-white color |
| Comparative Example 2 | HCl | 99.2 → 98.1 | Discolored to an off-white color |
| Example 6 | HCl | 99.5 → 99.2 | Color maintained |
| Example 7 | H₂SO₄ | 99.3 → 98.6 | Color maintained |
| Example 8 | HBr | 99.2 → 99.0 | Color maintained |
| Example 9 | HNO₃ | 98.2 → 97.7 | Color maintained |
| Example 10 | Acetic acid | 99.6 → 99.3 | Color maintained |
| Example 11 | Trifluoro acetic acid | 99.0 → 98.5 | Color maintained |
| Example 12 | Oxalic acid | 99.1 → 98.6 | Color maintained |
| Example 13 | Fumaric acid | 99.0 → 98.5 | Color maintained |
| Example 14 | Maleic acid | 99.1 → 98.5 | Color maintained |
| Example 15 | Citric acid | 99.0 → 98.6 | Color maintained |
| Example 16 | Methane sulfonic acid | 98.0 → 98.0 | Color maintained |

While the (R,S)-viloxazine hydrochloride of Comparative Example 1 and the (R)-viloxazine hydrochloride of Comparative Example 2 were discolored to an off-white color, the novel salts of (S)-viloxazine of Examples 6 to 16 maintained their color. In addition, the change in content of the novel salts of (S)-viloxazine of Examples 6 to 16 was minimal. Therefore, it was confirmed that the novel salts of (S)-viloxazine of Examples 6 to 16 had superior stability compared to the (R,S)-viloxazine hydrochloride of Comparative Example 1 and the (R)-viloxazine hydrochloride of Comparative Example 2.

## Claims

1. A method for preparing a compound of Formula 1 or a salt thereof, the method comprising:
reacting a compound of Formula 2 with a compound of Formula 3 in the presence of a base and a solvent (Step A-1); and
reacting a compound of Formula 4 obtained from Step A-1 with a compound of Formula 5 in the presence of a base and a solvent (Step A-2), 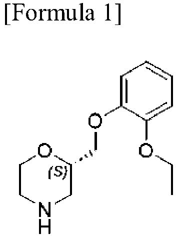 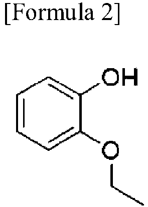 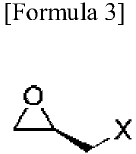 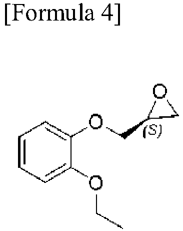 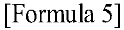 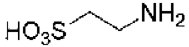
wherein X is Cl, Br, I, OMs (O-methane sulfonyl), or OTs (O-Tosyl) in Formula 3.

2. A method for preparing a compound of Formula 1 or a salt thereof, the method comprising: (i) reacting a compound of Formula 2 with a compound of Formula 6 in the presence of a solvent (Step B-1) or (ii) reacting a compound of Formula 2 with a compound of Formula 7 in the presence of a base and a solvent (Step B-2); and
deprotecting a compound of Formula 8 obtained from Step B-1 or Step B-2 (Step B-3), 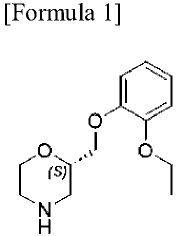 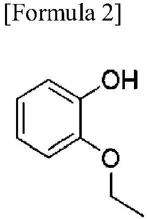 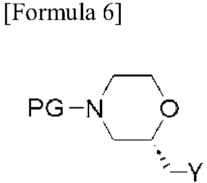 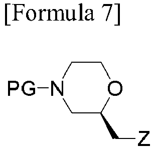 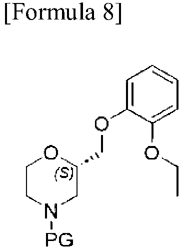
wherein PG is a protecting group in Formulas 6 to 8,
Y is OH in Formula 6, and
Z is Cl, Br, I, OMs (O-methane sulfonyl), or OTs (O-Tosyl) in Formula 7.

3. The method of claim 1, wherein the compound of Formula 3 in Step A-1 is used in an amount of 0.90 to 2.0 equivalents relative to the compound of Formula 2.

4. The method of claim 1, wherein the base in Step A-1 is at least one selected from the group consisting of sodium hydroxide, potassium hydroxide, calcium hydroxide, triethylamine, diethylamine, sodium carbonate, potassium carbonate, sodium bicarbonate, potassium bicarbonate, sodium t-butoxide, potassium t-butoxide, sodium hydride, sodium acetate, potassium acetate, and mixtures thereof.

5. The method of claim 1, wherein the base in Step A-1 is used in an amount of 0.9 to 2.0 equivalents relative to the compound of Formula 2.

6. The method of claim 1, wherein the solvent in Step A-1 is at least one selected from the group consisting of ethyl acetate, dichloromethane, toluene, methyl-t-butyl ether, acetone, tetrahydrofuran, dimethylformamide, N-methylpyrrolidone, dimethylacetamide, acetonitrile, and diethyl ether.

7. The method of claim 1, wherein in Step A-1, the solvent is used in a weight amount of 6 to 20 times the weight of the compound of Formula 2.

8. The method of claim 1, wherein in Step A-2, the base is at least one selected from the group consisting of sodium hydroxide, potassium hydroxide, calcium hydroxide, triethylamine, diethylamine, sodium carbonate, potassium carbonate, sodium bicarbonate, potassium bicarbonate, sodium t-butoxide, potassium t-butoxide, sodium hydride, sodium acetate, potassium acetate, and mixtures thereof.

9. The method of claim 1, wherein in Step A-2, the solvent is at least one selected from the group consisting of purified water, methanol, ethanol, isopropanol, butanol, and hexanol.

10. The method of claim 1, wherein in Step A-2, the solvent is used in a weight amount of 6 to 20 times the weight of the compound of Formula 5.

11. The method of claim 2, wherein in Step B-1 and Step B-2, the protecting group is tert-butoxycarbonyl (Boc), benzyl carbonyl (CBz), 9-fluorenylmethoxycarbonyl (Fmoc), or benzyl (Bn).

12. The method of claim 2, wherein in Step B-1, the solvent is at least one selected from the group consisting of tetrahydrofuran, benzene, dichloromethane, toluene, xylene, acetonitrile, and ethyl acetate.

13. The method of claim 2, wherein in Step B-2, the base is at least one selected from the group consisting of sodium hydroxide, potassium hydroxide, calcium hydroxide, triethylamine, diethylamine, sodium carbonate, potassium carbonate, sodium bicarbonate, potassium bicarbonate, sodium t-butoxide, potassium t-butoxide, sodium hydride, sodium acetate, potassium acetate, and mixtures thereof.

14. The method of claim 2, wherein the solvent in Step B-2 is at least one selected from the group consisting of ethyl acetate, dichloromethane, toluene, methyl-t-butyl ether, acetone, tetrahydrofuran, dimethylformamide, N-methylpyrrolidone, dimethylacetamide, acetonitrile, and diethyl ether.

15. The method of claim 1 or 2, further comprising reacting the prepared compound of Formula 1 with an acid in a solvent to form a salt of the compound of Formula 1.

16. The method of claim 15, wherein the solvent is at least one selected from the group consisting of ethyl acetate, dichloromethane, toluene, methyl-t-butyl ether, acetone, tetrahydrofuran, n-heptane, and diethyl ether.

17. The method of claim 15, wherein the acid is at least one selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methanesulfonic acid, ethanesulfonic acid, benzenesulfonic acid, and toluenesulfonic acid.

18. The method of claim 15, wherein the acid is hydrochloric acid or acetic acid.

19. The method of claim 15, wherein the salt of the compound of Formula 1 obtained is any one selected from the group consisting of (S)-viloxazine hydrochloride, (S)-viloxazine hydrobromide, (S)-viloxazine sulfate, (S)-viloxazine phosphate, (S)-viloxazine nitrate, (S)-viloxazine acetate, (S)-viloxazine glycolate, (S)-viloxazine lactate, (S)-viloxazine pyruvate, (S)-viloxazine malonate, (S)-viloxazine succinate, (S)-viloxazine glutarate, (S)-viloxazine fumarate, (S)-viloxazine malate, (S)-viloxazine mandelate, (S)-viloxazine tartrate, (S)-viloxazine citrate, (S)-viloxazine ascorbate, (S)-viloxazine palmitate, (S)-viloxazine maleate, (S)-viloxazine benzoate, (S)-viloxazine hydroxybenzoate, (S)-viloxazine phenylacetate, (S)-viloxazine cinnamate, (S)-viloxazine salicylate, (S)-viloxazine methanesulfonate, (S)-viloxazine ethanesulfonate, (S)-viloxazine benzenesulfonate, and (S)-viloxazine toluenesulfonate.

20. The method of claim 15, wherein the salt of the compound of Formula 1 obtained is (S)-viloxazine hydrochloride or (S)-viloxazine acetate.

21. A pharmaceutical composition for treating or preventing attention-deficit/hyperactivity disorder (ADHD), comprising the compound of Formula 1 or a salt thereof prepared according to claim 1, 2, or 15.

22. The pharmaceutical composition of claim 21, wherein the pharmaceutical composition is administered by a route of oral administration, parenteral administration, or topical administration.

23. A pharmaceutical composition for treating or preventing attention-deficit/hyperactivity disorder (ADHD), comprising (S)-viloxazine acetate.

24. A method for preventing or treating attention-deficit/hyperactivity disorder in a subject, the method comprising administering to the subject the compound of Formula 1 or a salt thereof prepared according to claim 1, 2, or 15.

25. Use of a compound of Formula 1 or a salt thereof prepared according to claim 1, 2, or 15 for the preparation of a medicament for preventing or treating attention-deficit/hyperactivity disorder.

26. Use of a compound of Formula 1 or a salt thereof prepared according to claim 1, 2, or 15 for preventing or treating attention-deficit/hyperactivity disorder.

27. A method for preventing or treating attention-deficit/hyperactivity disorder in a subject, the method comprising administering (S)-viloxazine acetate to the subject.

28. Use of (S)-viloxazine acetate for the preparation of a medicament for preventing or treating attention-deficit/hyperactivity disorder.

29. Use of (S)-viloxazine acetate for preventing or treating attention-deficit/hyperactivity disorder.
